# EUROPEAN PATENT APPLICATION

(11) **EP 2 821 001 A1**
(43) Date of publication of application: **07.01.2015**
(21) Application number: 13823156.8
(22) Date of filing: 12.06.2013
(51) Int. Cl.: A61B 1/00, A61B 19/00

(54) **PROCEDURAL INSTRUMENT INSERTION ASSISTANCE INSTRUMENT**

(30) Priority: 26.07.2012 JP 2012166132
(71) Applicant: Olympus Medical Systems Corp., Tokyo 151-0072 (JP)
(72) Inventor: NISHINA, Kenichi, Tokyo 150-0072 (JP); HASHIGUCHI, Toshihiko, Tokyo 150-0072 (JP); KAWASHIMA, Tomonao, Tokyo 150-0072 (JP)
(74) Representative: Gunzelmann, Rainer
(86) International application number: PCT/JP2013/066224
(87) International publication number: WO 2014/017205

(57) **Abstract**

A treatment instrument insertion auxiliary is used to subserve passage of an elongated treatment instrument into a treatment instrument channel by being connected to an endoscope provided with a rigid insertion portion which includes the treatment instrument channel adapted to allow passage of the treatment instrument and a treatment instrument channel opening portion formed at a proximal end of the treatment instrument channel and used to insert the treatment instrument, where the treatment instrument insertion auxiliary includes a treatment instrument guide unit adapted to guide a distal end side of the treatment instrument in a central axis direction of the treatment instrument channel opening portion by being placed in contact with the distal end side of the treatment instrument.

## Description

### Technical Field

The present invention relates to a treatment instrument insertion auxiliary adapted to subserve insertion of a treatment instrument into a treatment instrument channel provided in an endoscope equipped with a rigid insertion portion.

### Background Art

Recently, endoscopes have come to be used widely to examine affected areas and the like in a body. Also, in order to be able to deal with a situation in which it is necessary to treat an affected area, the endoscope is provided with a treatment instrument channel which allows passage of a treatment instrument. Then, a surgeon can pass the treatment instrument through the treatment instrument channel through an opening portion at a proximal end of the treatment instrument channel and cause the treatment instrument to protrude from a distal end opening of the treatment instrument channel to perform a treatment, such as a biopsy, of the affected area.

The opening portion at the proximal end of the treatment instrument channel is small in size and formed in a predetermined direction so as to point to the side of an insertion portion. Consequently, it sometimes requires skill to smoothly insert a treatment instrument with a sharp distal end shape such as a needle shape without causing damage to an inner wall of the treatment instrument channel and without causing damage to the sharp distal end shape of the treatment instrument.

Thus, a treatment instrument insertion auxiliary is sometimes used to subserve an operation of smoothly inserting a treatment instrument into the treatment instrument channel through the opening portion at the proximal end of the treatment instrument channel.

As a conventional example, for example, Japanese Patent Application Laid-Open Publication No. 11-225950 discloses a treatment instrument insertion auxiliary wherein a flexible tube bridges between a treatment instrument inlet provided in a hand side end portion and used to insert a treatment instrument and an endoscope connection portion provided at a distal end to connect to a treatment instrument insertion port of an endoscope; and a slit is formed continuously from the treatment instrument inlet to at least a location in a neighborhood of the endoscope connection portion of the flexible tube.

However, the conventional example described above assumes that a flexible treatment instrument is passed through a treatment instrument channel provided in a flexible insertion portion, making it difficult to efficiently subserve insertion into a small inner diameter treatment instrument channel provided in a rigid insertion portion long in total length so as to allow passage of a rigid treatment instrument, such as a puncture needle, with a small outside diameter and a long total length.

With a treatment instrument, such as a puncture needle, low in flexibility, small in outside diameter, and long in total length in this way, when a proximal end of the treatment instrument is gripped in an attempt to insert a distal end of the treatment instrument into an insertion port (opening portion) of the treatment instrument channel near a rear end of the endoscope, the treatment instrument becomes prone to generate or induce vibration due to (unintentional) shaking of the hand.

More specifically, when one surgeon attempts to insert the distal end of the treatment instrument into the insertion port of the treatment instrument channel near the rear end of the endoscope by gripping a grasping portion of the endoscope with one hand and gripping the proximal end of the treatment instrument with the other hand, since the proximal end of the treatment instrument is gripped in a cantilevered fashion, a distal end side of the treatment instrument becomes prone to vibrate as if in resonance with light shaking of a proximal end side of the treatment instrument, and the vibration of the distal end side of the treatment instrument makes the operation of inserting the treatment instrument into the small insertion port of the treatment instrument channel in a short time very difficult.

Insertion of such a treatment instrument into the insertion port in a short time requires not only one surgeon, but also an assistant who assists an operation of insertion into the insertion port of the treatment instrument channel, for example, by gripping the distal end of the treatment instrument. Note that in order to carry out a biopsy using a treatment instrument for biopsy, it is normally necessary to repeat similar operations at plural different sites.

Therefore, there is demand for a treatment instrument insertion auxiliary equipped with a function to guide a distal end side of a treatment instrument smoothly in a central axis direction near an insertion opening portion of the treatment instrument channel for a single surgeon without requiring an assistant.

The present invention has been made in view of the above points and has an object to provide a treatment instrument insertion auxiliary which can smoothly guide a distal end side of a treatment instrument in a central axis direction of an insertion opening portion of a treatment instrument channel.

### Disclosure of Invention

### Means for Solving the Problem

One aspect of the present invention provides a treatment instrument insertion auxiliary used to subserve passage of an elongated treatment instrument into a treatment instrument channel by being connected to an endoscope provided with a rigid insertion portion which includes the treatment instrument channel formed along an axis and adapted to allow passage of the treatment instrument and a treatment instrument channel opening portion formed at a proximal end of the treatment instrument channel and used to insert the treatment instrument, the treatment instrument insertion auxiliary comprising a treatment instrument guide unit adapted to guide a distal end side of the treatment instrument in a central axis direction of the treatment instrument channel opening portion by being placed in contact with the distal end side of the treatment instrument.

### Brief Description of the Drawings

Fig. 1 is a diagram showing an overall configuration of an ultrasound endoscope apparatus equipped with a first embodiment of the present invention.
Fig. 2 is a diagram showing a distal end face in an attached state in which an optical scope is attached to an ultrasound probe and showing a relationship in which an observation field of view of an objective lens 21 and a center of a channel distal end opening are located on a scanning plane of an ultrasound transducer.
Fig. 3 is a diagram showing a configuration of a puncture needle apparatus as a treatment instrument.
Fig. 4 is a sectional view showing a configuration of an adapter member detachably connected to a treatment instrument channel.
Figs. 5(A) to 5(C) are diagrams showing a configuration of a treatment instrument insertion auxiliary.
Fig. 6 is a diagram showing an unprocessed shape of a flat plate used to produce the treatment instrument insertion auxiliary.
Fig. 7 is a flowchart of operation including details of a treatment instrument insertion method according to the first embodiment.
Figs. 8(A) to 8(C) are explanatory diagrams of an operation of inserting a puncture needle using the treatment instrument insertion auxiliary.
Fig. 9 is a diagram showing a distal end of the puncture needle and a protruding length of an automatic biopsy on an ultrasound tomographic image with a distal end of an outer tube fixed at a position near α on an inner tube with a fixing screw.
Fig. 10 is a diagram showing the distal end of the puncture needle and the protruding length of the automatic biopsy on an ultrasound tomographic image with the distal end of the outer tube fixed at a position near β on the inner tube with the fixing screw.
Fig. 11 is a diagram showing the distal end of the puncture needle and the protruding length of the automatic biopsy on an ultrasound tomographic image with the distal end of the outer tube fixed at a position near γ on the inner tube with the fixing screw.
Fig. 12 is a perspective view showing an appearance of a treatment instrument insertion auxiliary according to a second embodiment of the present invention.
Fig. 13 is a perspective view showing the treatment instrument insertion auxiliary according to the second embodiment in partial cutaway fashion.
Fig. 14 is a perspective view showing a treatment instrument insertion auxiliary according to a variation of the second embodiment.
Fig. 15 is a perspective view showing a schematic configuration of a treatment instrument insertion auxiliary according to a third embodiment of the present invention.
Fig. 16 is a rear view of the treatment instrument insertion auxiliary according to the third embodiment mounted on a channel tube.
Fig. 17 is a perspective view showing a schematic configuration of a treatment instrument insertion auxiliary according to a variation of the third embodiment.
Fig. 18 is a rear view of the treatment instrument insertion auxiliary of Fig. 17.

### Best Mode for Carrying Out the Invention

Embodiments of the present invention will be described below with reference to the drawings.

### (First Embodiment)

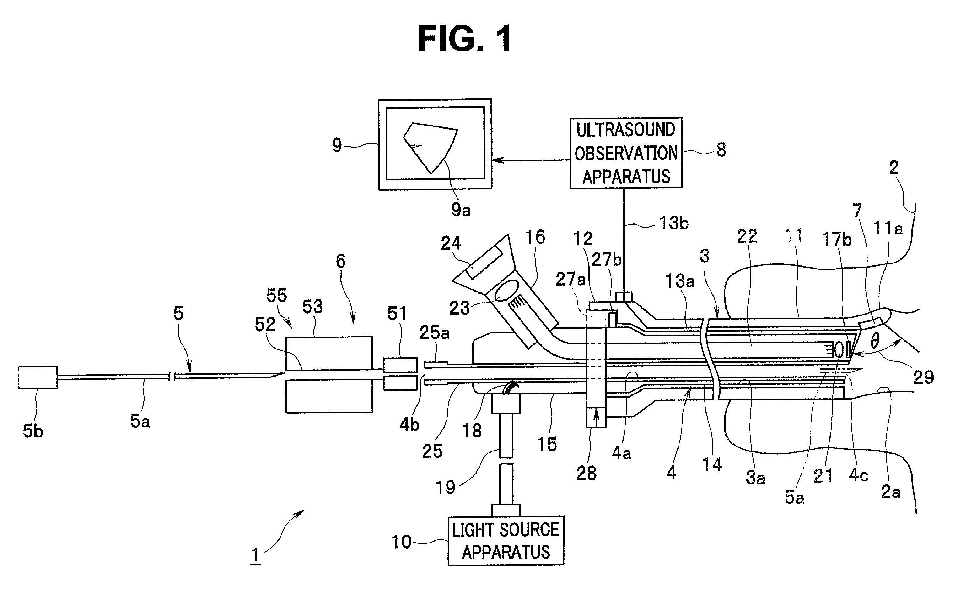

Fig. 1 shows an ultrasound endoscope apparatus 1 equipped with a treatment instrument insertion auxiliary 6 according to a first embodiment of the present invention.

The ultrasound endoscope apparatus 1 includes a tube-shaped ultrasound probe 3 inserted into, for example, the urethra 2a of a patient 2, an optical scope 4 as a rigid endoscope passed through an optical scope passage channel (also referred to simply as a channel) 3a of the ultrasound probe 3, a puncture needle apparatus 5 as a treatment instrument inserted into a treatment instrument channel (also referred to simply as a channel) 4a provided in the optical scope 4, and the treatment instrument insertion auxiliary 6 used to subserve in inserting a puncture needle 5a of the puncture needle apparatus 5 into the channel 4a.

Also, the ultrasound endoscope apparatus 1 includes an ultrasound observation apparatus 8 adapted to generate an ultrasound tomographic image by performing signal processing using an ultrasound transducer 7 provided on the ultrasound probe 3, a monitor 9 as display means of displaying the generated ultrasound tomographic image, and a light source apparatus 10 adapted to supply illuminating light to the optical scope 4.

The ultrasound probe 3 includes a rigid probe insertion portion 11 tubular in shape, and a probe grasping portion 12 provided at a rear end (proximal end) of the probe insertion portion 11 by being expanded in diameter. A distal end portion 11a of the probe insertion portion 11 protrudes with some part in a circumferential direction bending backward and the ultrasound transducer 7 of a convex type is provided along a convex surface on an inner circumferential face side of the protruding distal end portion 11 a.

The ultrasound transducer 7 is connected to the ultrasound observation apparatus 8 via a signal cable 13a passed through the probe insertion portion 11 and a signal cable 13b connected to a connector of the probe grasping portion 12 at the rear end of the probe insertion portion 11.

The ultrasound observation apparatus 8 applies an ultrasound drive signal to the ultrasound transducer 7 via signal cables 13a and 13b, driving the ultrasound transducer 7 so as to transmit ultrasound as well as generating an ultrasound tomographic image from an ultrasound signal acquired by the ultrasound transducer 7.

The optical scope 4 includes a rigid insertion portion 14 and a grasping portion 15 provided at a rear end of the insertion portion 14 by being expanded in diameter. An eyepiece barrel 16 is provided near a rear end of the grasping portion 15.

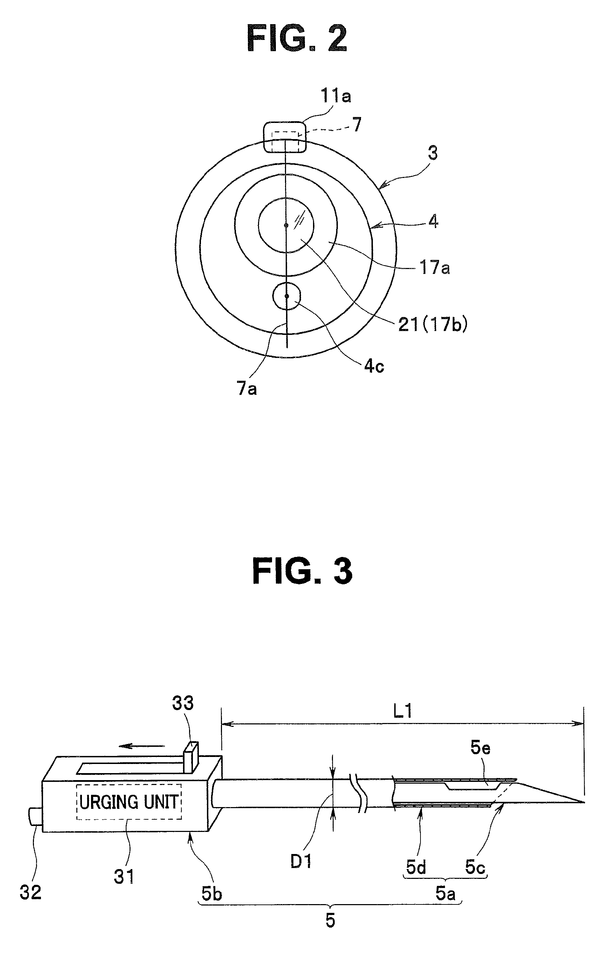

The optical scope 4 includes an illuminating window 17a (see Fig. 2) and observation window 17b provided in a distal end face of a distal end portion of the insertion portion 14. A light guide 18 (only part of which on a rear end side is shown in Fig. 1) adapted to transmit illuminating light is passed through the insertion portion 14 and the grasping portion 15 and a rear end of the light guide 18 is connected to the light source apparatus 10 via a light guide cable 19 connected to a connector on a flank of the grasping portion 15.

The illuminating light generated by the light source apparatus 10 is emitted forward of the illuminating window 17a via the light guide cable 19 and the light guide 18 and through the illuminating window 17a on which a distal end face of the light guide 18 is placed.

An objective lens 21 is placed in the observation window 17b provided adjacent to the illuminating window 17a (on a center side of the illuminating window 17a, according to the present embodiment) and adapted to form an optical image of an object such as an affected area illuminated by illuminating light emitted from the illuminating window 17a.

A distal end face of an image guide fiber bundle 22 serving as image-forming transmission means passed through the insertion portion 14 is placed at an image location of the objective lens 21, and the optical image of the object formed on the distal end face is transmitted to a rear end face by the image guide fiber bundle 22. A rear end side of the image guide fiber bundle 22 is extended from the middle of the grasping portion 15 toward the eyepiece barrel 16 and the rear end face is placed in the eyepiece barrel 16.

An eyepiece lens 23 is placed in the eyepiece barrel 16, facing the rear end face of the image guide fiber bundle 22, and the surgeon can observe the optical image of the object transmitted through the image guide fiber bundle 22, by viewing through an eyepiece window 24 provided at a rear end of the eyepiece barrel 16. Note that the eyepiece window 24 is covered by clear plate glass.

Also, the channel 4a is provided by a hollow portion formed along an axis penetrating through a distal end face of the insertion portion 14 and a rear end face of the grasping portion 15 along an axial direction of the insertion portion 14 and the grasping portion 15 of the optical scope 4. The channel 4a is made up of a hollow portion of a channel tube 25 cylindrical in shape and in a configuration in Fig. 1, a rear end side of the channel tube 25 protrudes further rearward from the rear end face of the grasping portion 15. A rear end of the channel tube 25 protruding rearward from the rear end face of the grasping portion 15 opens as a channel opening portion (or a treatment instrument insertion port) 4b of the channel 4a. A connection portion 51 provided at a distal end of the treatment instrument insertion auxiliary 6 is detachably connected to (mounted on) the channel opening portion 4b.

Also, a distal end of the channel 4a opens as a channel distal end opening portion (or a treatment instrument projection port) 4c. Note that, for example, a connecting tube 25a increased in thickness in a step-like manner is formed near the rear end of the channel tube 25 near the channel opening portion 4b and is detachably connected with the connection portion 51 of the treatment instrument insertion auxiliary 6.

In an inner circumferential face of the probe grasping portion 12 on a rear end side of the ultrasound probe 3, a grooved portion 27a used for circumferential positioning of the optical scope 4 passed through the channel 3a is formed to an appropriate depth from a rear end of the inner circumferential face of the probe grasping portion 12. On the other hand, a projection 27b configured to fit in the grooved portion 27a is provided at a predetermined location on an outer circumferential face of the optical scope 4 near a front end of the grasping portion 15.

Then, the circumferential positioning of the optical scope 4 passed through the channel 3 a of the ultrasound probe 3 is done by the grooved portion 27a and the projection 27b to allow the optical scope 4 to be attached to inside the channel 3a of the ultrasound probe 3 in a predetermined state of passage.

Also, a lock member 28 is provided near a rear end of the probe grasping portion 12 to restrict (block) rearward movement of the projection 27b fitted in the grooved portion 27a.

Then, for example, as shown in Fig. 1, with the projection 27b fitted in the grooved portion 27a, when the lock member 28 is moved up as indicated by an arrow (moved positions are indicated by dotted lines), the optical scope 4 can be locked in a predetermined attached state by restricting the (rearward) movement of the projection 27b. In the attached state, the ultrasound probe 3 and the optical scope 4 are fixed to each other, forming an integrated whole.

In the attached state, the ultrasound transducer 7 sends and receives ultrasound within a fan-shaped angle θ in the plane of the paper, for example, shown in Fig. 1, forming an ultrasound scan field 29 in which ultrasound observation is possible.

Note that on a distal end side of the ultrasound probe 3, for example, plural ultrasound transducer elements are arranged along a longitudinal direction of the distal end portion 11a in a convex portion on the side of the channel 3a, forming the convex ultrasound transducer 7. Then, the ultrasound observation apparatus 8 generates an ultrasound tomographic image corresponding to the ultrasound scan field 29 from an ultrasound signal acquired by the convex ultrasound transducer 7 and displays an ultrasound tomographic image 9a, for example, on a display surface of the monitor 9 shown in Fig. 1.

Also, as shown in Fig. 1, the ultrasound scan field 29 is set to cover a fan-shaped range from near a distal end face of the optical scope 4 to a forward side of the distal end face.

Therefore, in Fig. 1, a distal end side of the puncture needle 5a can be captured in the ultrasound scan field 29 and viewed on the ultrasound tomographic image 9a not only when the distal end side of the puncture needle 5a is set to a state of protruding slightly from the channel distal end opening portion 4c of the optical scope 4 as indicated by a chain double-dashed line, but also when the distal end side of the puncture needle 5a protrudes further forward from a position of the chain double-dashed line.

Fig. 2 shows a front view from the distal end side of the ultrasound probe 3 in an attached state in which the optical scope 4 is attached to the ultrasound probe 3, and shows a relationship in which an observation field of view of the objective lens 21 and a center of the channel distal end opening portion 4c are located on a scanning plane of the ultrasound transducer 7.

As shown in Fig. 2, the center of the observation field of view of the objective lens 21 and the center of the channel distal end opening portion 4c are set to be located on the scanning plane (scanning center plane) 7a in which ultrasound is emitted (scanned) fanwise by the ultrasound transducer 7 provided at a distal end of the ultrasound probe 3. Note that a center of the channel 4a also coincides with a center of the channel distal end opening portion 4c. This allows the distal end side of the puncture needle 5a passing through the channel 4a and protruding from the channel distal end opening portion 4c to be captured in the ultrasound scan field 29 as described above.

Also, as shown in Fig. 3, the puncture needle apparatus 5 includes the elongated rigid puncture needle 5a and an operation portion (or grasping portion) 5b provided at a rear end of the puncture needle 5a. The puncture needle 5a includes a puncture inner needle (inner needle) 5c and a puncture outer needle tube (outer needle tube) 5d through which the inner needle 5c is movably passed. Note that the puncture needle apparatus 5 is a disposable puncture needle apparatus and is discarded once used for tissue sampling (biopsy).

Also, the puncture needle 5a has an outside diameter D1 smaller than 2 mm and a total length L1 of about 400 mm. In this way, the puncture needle 5a is small in outside diameter D 1 and very long in total length L1. Therefore, the inner needle 5c and the outer needle tube 5d are formed of rigid material such as stainless steel, and the distal end side of the puncture needle 5a becomes prone to induce vibration.

As shown in Fig. 3, in the puncture needle 5a, normally that part of the inner needle 5c which is located rearward of a proximal end of a cutting edge truncated diagonally is housed inside a distal end opening of the outer needle tube 5d, for example, truncated diagonally. Also, by cutting away part of an outer circumferential face, a recess 5e serving as a tissue housing portion is provided near a distal end of the inner needle 5c to house biopsied tissue. The recess 5e is normally retracted in the distal end opening of the outer needle tube 5d.

Also, the grasping portion 5b contains an urging unit 31 and a trigger button 32, where the urging unit 31 urges the inner needle 5c and outer needle tube 5d so as to protrude forward while the trigger button 32 is operated ON/OFF to unleash the urging unit 31.

When the surgeon pushes the trigger button 32, the urging unit 31 is set off and causes the inner needle 5c or outer needle tube 5d to protrude rapidly.

More specifically, when the trigger button 32 is operated the first time, the inner needle 5c protrudes, and when the trigger button 32 is operated the second time, the outer needle tube 5d protrudes to get ready to sample, i.e., to biopsy the tissue in the recess 5e. (Hereinafter referred to as "automatic biopsy.")

After the protrusion of the outer needle tube 5d, when the surgeon pulls a non-illustrated urging lever, the urging unit 31 gets ready to urge the inner needle 5c and the outer needle tube 5d again.

Also, the grasping portion 5b is provided with a sampling lever 33 to collect biopsied tissue, and the sampling lever 33 is configured to be slidable in a longitudinal direction as shown in Fig. 3. Then, after the urging is released, when the surgeon slides the sampling lever 33 rearward, the outer needle tube 5d moves rearward as well, revealing the recess 5e provided near the distal end of the inner needle 5c.

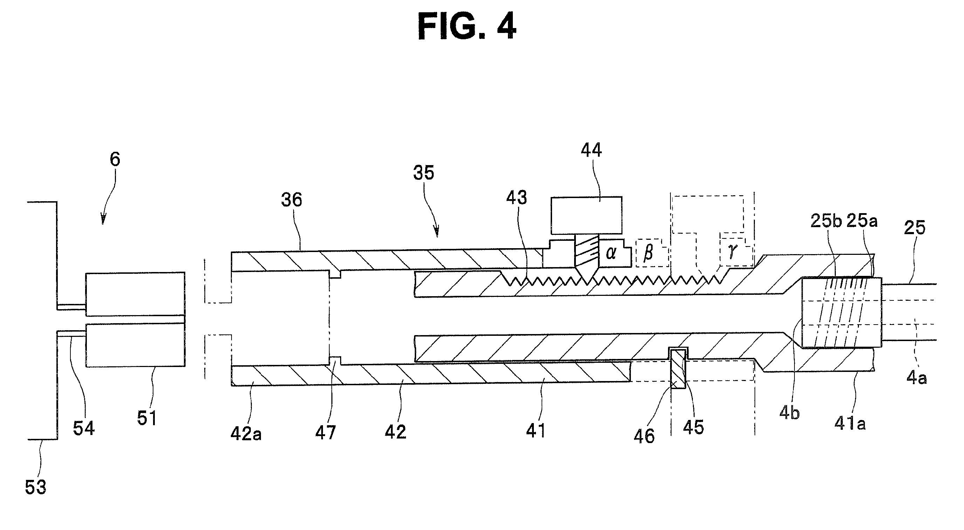

Also, in an exemplary configuration shown in Fig. 1, the present ultrasound endoscope apparatus 1 is configured such that the connection portion 51 at the distal end of the treatment instrument insertion auxiliary 6 can be attached to the channel opening portion 4b of the optical scope 4 to allow the distal end side of the puncture needle 5a (whose insertion is to be subserved) to be inserted into the treatment instrument insertion auxiliary 6 from behind. This configuration is not restrictive, and an adapter member (or adapter unit) 35 equipped with a slide unit slidable along an axial direction of the channel 4a of the optical scope 4 may be connected to the channel opening portion 4b of the optical scope 4 as shown in Fig. 4, allowing the treatment instrument insertion auxiliary 6 to be attached to a rear end of the adapter member 35. Note that although according to the present embodiment, the adapter member (or adapter unit) 35 is detachably mounted to the treatment instrument insertion auxiliary 6, the adapter member 35 may be mounted integrally to the treatment instrument insertion auxiliary 6.

When the adapter member 35 is interposed, it becomes not only easy to perform a biopsy operation using the puncture needle 5a under ultrasound observation by means of the ultrasound probe 3, but also possible to make adjustments to obtain a length convenient for biopsy by compensating for differences in the length of the puncture needle 5a regardless of whether ultrasound observations are carried out using the ultrasound probe 3.

Thus, the treatment instrument insertion auxiliary 6 according to the present embodiment can be used to subserve insertion of the elongated puncture needle 5a of the puncture needle apparatus 5 serving as the treatment instrument by being connected to the channel opening portion 4b of the optical scope 4 as shown in Fig. 1 with the optical scope 4 serving as a rigid endoscope as well as used to subserve insertion of the puncture needle 5a of the puncture needle apparatus 5 by being connected to the rear end of the adapter member 35 connected to the channel opening portion 4b as shown in Fig. 4.

Therefore, by regarding the adapter member 35 as a component of the treatment instrument insertion auxiliary 6, the treatment instrument insertion auxiliary 6 can be defined as being equipped with the adapter member (or adapter unit) 35 which in turn is equipped with a slide unit slidable along the axial direction of the channel 4a of the rigid endoscope and variable in total length. Note that the adapter member 35 is a reusable member (capable of being used repeatedly).

The adapter member 35 includes an inner tube 41 provided with a first connection portion 41a whose front end is detachably connected to the connecting tube 25a provided with the channel opening portion 4b, an outer tube 42 fitted with the inner tube 41 to become slidable and functioning as a slide unit variable in total length, where a second connection portion 42a at a rear end of the outer tube 42 is detachably connected with the connection portion 51 at the front end (distal end) of the treatment instrument insertion auxiliary 6.

Note that as indicated by chain double-dashed lines in Fig. 4, a male thread portion 25b and a female thread portion designed to screw together may be provided on an outer circumferential face of the connecting tube 25a and an inner circumferential face of the first connection portion 41a, respectively, to allow the connecting tube 25a and the first connection portion 41a to be detachably connected. In this case, a female thread portion designed to screw onto the male thread portion 25b may also be provided in an inner circumferential face of the connection portion 51 at the front end of the treatment instrument insertion auxiliary 6 to detachably connect the connection portion 51 of the treatment instrument insertion auxiliary 6 to the connecting tube 25a by screwing if the adapter member 35 is not interposed.

Also, the inner tube 41 of the adapter member 35 has a concavo-convex portion 43 formed spirally to a predetermined length on an outer circumferential face excluding opposite ends of the inner tube 41 such that a distal end of a fixing screw 44 provided near a distal end of the outer tube 42 will be inserted and engaged with the concavo-convex portion 43 shaped like triangular waves, to allow the slidable outer tube 42 to be fixed to the inner tube 41.

Also, a circumferential groove 45 is formed in the outer circumferential face of the inner tube 41 at a predetermined location in a length direction, allowing a movement restriction member 46 in the form of a C-shaped ring to be inserted and engaged with the circumferential groove 45.

The distal end of the fixing screw 44 can be pressed against the triangular wave-shaped concavo-convex portion 43 of the inner tube 41 by penetrating an inner side of the outer tube 42 from an outer side. A head of the fixing screw 44 is pointed in the shape of a triangular pyramid. When a user such as the surgeon tightens the fixing screw 44, the distal end of the fixing screw 44 is pressed against the concavo-convex portion 43 of the inner tube 41. Consequently, the outer tube 42 is fixed to the inner tube 41 so as not to move in the axial direction. Solid lines in Fig. 4 indicate a state in which the distal end of the outer tube 42 is fixed at position α on the inner tube 41.

When the fixing screw 44 is loosened, the distal end of the fixing screw 44 is no longer pressed against the concavo-convex portion 43 of the inner tube 41, and the outer tube 42 becomes movable again in the axial direction relative to the inner tube 41.

When the movement restriction member 46 removably inserted into the circumferential groove 45 of the inner tube 41 is fitted in the circumferential groove 45, (the distal end of) the outer tube 42 can move to position β in Fig. 4. When the movement restriction member 46 is not fitted in the circumferential groove 45, the outer tube 42 can move to position γ in Fig. 4.

A projection 47 is provided on an inner circumferential face at the rear end of the outer tube 42 such that the connection portion 51 will abut against the projection 47 to keep a press-fitting length when the distal end of the connection portion 51 of the treatment instrument insertion auxiliary 6 is press-fitted as indicated by chain double-dashed lines.

Note that according to the present embodiment, when the adapter member 35 is used, the connection portion 51 of the treatment instrument insertion auxiliary 6 is detachably connected by being press-fitted in the outer tube 42. In contrast, when the adapter member 35 is not used, the connection portion 51 of the treatment instrument insertion auxiliary 6 is detachably connected by being fitted over the connecting tube 25a of the channel tube 25.

The connection portion 51 of the treatment instrument insertion auxiliary 6 is made of a cylindrical member formed into a C-shaped ring with a cut (notch) provided in its longitudinal direction so that the connection portion 51 can be detachably connected by absorbing slight radial dispersion in inner diameter or outside diameter size of the connection portion 51 (or an object to which the connection portion 51 is connected).

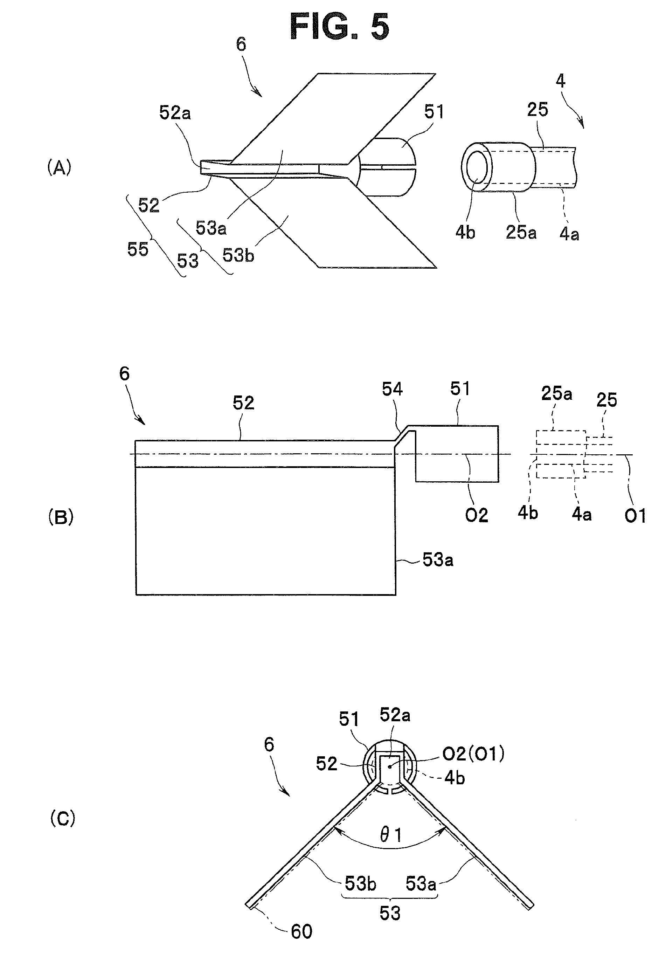

Next, a configuration of the treatment instrument insertion auxiliary 6 according to the first embodiment will be described with reference to Fig. 5 and the like.

Fig. 5(A) shows a perspective view of the treatment instrument insertion auxiliary 6 detachably connected to the channel opening portion 4b of the optical scope 4, for example, shown in Fig. 1. Fig. 5(B) shows a plan view of the treatment instrument insertion auxiliary 6 as viewed from above in the plane of the paper in Fig. 5(A). Fig. 5(C) shows a rear view of the treatment instrument insertion auxiliary 6 as viewed from the left side in Fig. 5(B) (with central axes 01 and 02 aligned with each other on a same axis).

As shown in Fig. 5, the treatment instrument insertion auxiliary 6 includes the connection portion (or an attaching portion) 51 formed as a C-shaped ring and detachably connected to (the connecting tube 25a of) the channel tube 25 at a proximal end of the optical scope 4 serving as an endoscope (rigid endoscope) having a rigid insertion portion; a guide path (or a guide unit) 52 installed consecutively at a rear end of the connection portion 51 via a coupling portion 54 and adapted to guide the distal end side of the puncture needle 5a toward the central axis 01 of the channel opening portion 4b; and a receiving portion 53 abutted by or placed in contact with the distal end side of the puncture needle 5a and adapted to suppress vibration and the like of the distal end side of the puncture needle 5a (which receives, or is placed in contact with, a contacting portion).

In this way, the treatment instrument insertion auxiliary 6 includes a treatment instrument guide unit 55 which in turn includes the receiving portion 53 adapted to suppress (or absorb) vibration and the like of the distal end side of the puncture needle 5a by being placed in contact with the distal end side of the puncture needle 5a, and the guide path 52 having a guide groove (guiding groove) 52a U-shaped in cross-section and adapted to guide the distal end side of the puncture needle 5a caught by the receiving portion 53 in a central axis direction of the channel opening portion 4b.

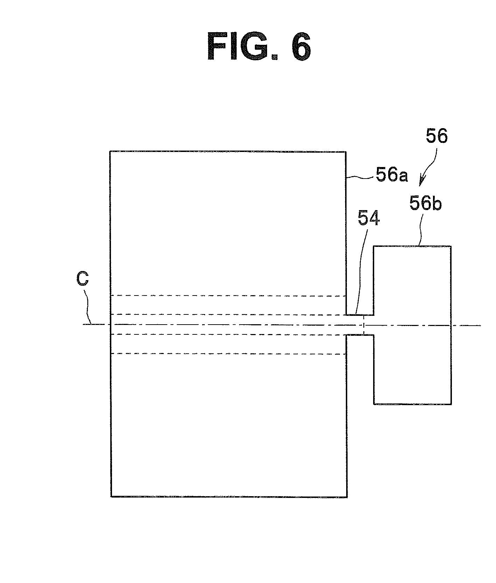

The guide path 52 and the plate members 53a and 53b are formed on the treatment instrument insertion auxiliary 6, where the guide path 52 includes the U-shaped guide groove 52a formed by bending the larger rectangular portion 56a of a flat plate 56 such as shown in Fig. 6 along dotted lines while the plate members 53a and 53b make up the receiving portion 53. Also, the connection portion 51 whose inner diameter is fitted in an outside diameter of the connecting tube 25a is formed by bending the smaller rectangular portion 56b coupled by the coupling portion 54 into a cylindrical shape (which is to become a C-shaped ring). Note that the flat plate 56 is shaped symmetrically in the vertical direction with respect to a horizontal center line C.

Also, as shown in Fig. 5(B), the coupling portion 54 is bent midway in a longitudinal direction such that the central axis 02 of the guide path 52 will coincide with a central axis of the connection portion 51.

Note that when the connection portion 51 is connected (attached) so as to fit in the connecting tube 25a, the central axis of the connection portion 51 (and the central axis 02 of the guide path 52) coincides with the central axis 01 of the channel 4a of the channel tube 25. In other words, the central axis of the connection portion 51 and the central axis 02 of the guide path 52 are concentric with (common to) the central axis 01 of the channel 4a or the channel opening portion 4b and are formed on an extension of the central axis 01.

When viewed from behind (from a rear end side of) the central axis 02, the treatment instrument insertion auxiliary 6 looks as shown in Fig. 5(C). Note that a dotted line indicates the channel opening portion 4b when the connection portion 51 is connected to the connecting tube 25a with the central axis 02 coinciding with (the extension of) the central axis 01.

Also, as shown in Fig. 5(C), the entire U-shaped guide groove 52a of the guide unit 52 is set to be located inside a circular opening of the channel opening portion 4b indicated by the dotted line. In other words, the guide path 52a includes a guiding abutment portion configured to be abuttable by the distal end side of the puncture needle 5a at at least two locations along a direction parallel to the central axis 01 (at plural locations on an inner surface of the guide groove 52a, according to the present embodiment) within a distance equal to a radius (inner radius) of the channel 4a or the channel opening portion 4b from the central axis 01 of the channel 4a or the channel opening portion 4b, and has a function of a guiding portion (guiding means) serving as a guide when a moving direction on a distal end side of the treatment instrument is a direction towards insertion into the channel opening portion 4b.

Therefore, by putting the distal end side of the puncture needle 5a into the guide groove 52a of the guide path 52 and moving the distal end side of the puncture needle 5a in a longitudinal direction of the guide groove 52a having a function of the guiding portion, the surgeon can easily insert the distal end side of the puncture needle 5a into the channel opening portion 4b. Note that since the cross-section (perpendicular to the longitudinal direction) of the guide groove 52a is U-shaped, the guide groove 52a functions as a restricting portion (guide unit) or restricting means (guiding means) which restricts (guides) the moving direction of the distal end side of the puncture needle 5a in the guide groove 52a to a direction of insertion into the channel opening portion 4b so as to coincide with a direction in which a U-shaped inner surface extends.

As can be seen from Fig. 5(C), the plate members 53a and 53b making up the receiving portion 53 is spread to a V-shape having an appropriate angle θ1 (about 90 degrees in the concrete example of Fig. 5(C)) and those one sides (upper sides in the state of Fig. 5(C)) of the plate members 53a and 53b which face each other in close vicinity form a U-shaped open end of the guide path 52. That is, the guide path 52 is open to a side (lower side in Fig. 5(C)) on which the paired plate members 53a and 53b face each other in close vicinity.

Thus, as described later, the surgeon can set the distal end side of the puncture needle 5a easily in the guide groove 52a of the guide path 52 by placing the distal end side of the puncture needle 5a in contact with planar portions of the plate members 53a and 53b which spread in a V-shape from opposite edges of the small opening portion of the guide path 52 and moving the distal end side of the puncture needle 5a placed in contact with the planar portions toward the guide path 52. Note that although in the present embodiment, the guide groove 52a has a U-shaped cross-section, the shape of the cross-section is not limited to a U-shape and may be, for example, a C-shape formed by cutting away part of a circular shape.

Thus, the treatment instrument insertion auxiliary 6 according to the present embodiment is a treatment instrument insertion auxiliary 6 used to subserve passage of an elongated treatment instrument into a treatment instrument channel by being connected to the optical scope 4 serving as an endoscope provided with the rigid insertion portion 14 which includes the channel 4a serving as the treatment instrument channel formed along an axis and adapted to allow passage of the puncture needle 5a of the puncture needle apparatus 5 serving as the treatment instrument and the channel opening portion 4b serving as a treatment instrument channel opening portion formed at a proximal end of the treatment instrument channel and used to insert the treatment instrument, the treatment instrument insertion auxiliary comprising the treatment instrument guide unit 55 adapted to guide a distal end side of the treatment instrument in a central axis direction of the treatment instrument channel opening portion by being placed in contact with the distal end side of the treatment instrument.

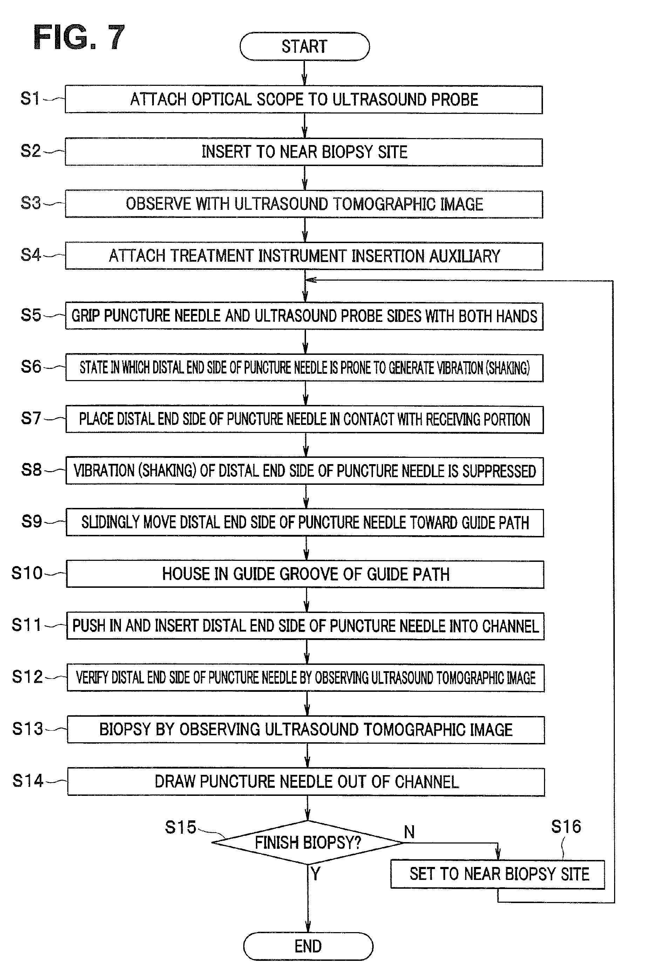

Next, operation of the present embodiment will be described. Fig. 7 shows a flowchart of operation including procedures of a treatment instrument insertion method according to the present embodiment.

To begin with, in step S 1 first, the surgeon passes the optical scope 4 through the channel 3a of the ultrasound probe 3 as shown in Fig. 1 and attaches the optical scope 4 to the ultrasound probe 3 by operating the lock member 28.

Next, in step S2, under optical observation through the eyepiece window 24 of the optical scope 4, the surgeon inserts the ultrasound probe 3 into, for example, the urethra 2a and sets the distal end portion 11a of the ultrasound probe 3 to near a biopsy site where a biopsy is going to be performed.

Also, as shown in step S3, by sending and receiving ultrasound to/from the side of the biopsy site using the ultrasound transducer 7 provided in the distal end portion 11a of the ultrasound probe 3, the surgeon creates conditions in which the biopsy site can be observed with the ultrasound tomographic image 9a.

Next, in step S4, the surgeon connects (attaches) the connection portion 51 of the treatment instrument insertion auxiliary 6 to the connecting tube 25a of the optical scope 4. Note that the treatment instrument insertion auxiliary 6 does not necessarily have to be connected (attached) in step S4, and may be connected (attached) in a step previous to step S4.

In the following description, it is assumed that the adapter member 35 is not used.

Next, in step S5, the surgeon grips the grasping portion 15 of the optical scope 4 or a peripheral portion of the probe grasping portion 12 of the ultrasound probe 3 with one hand and grips the grasping portion 5b of the puncture needle apparatus 5 with the other hand to perform tissue sampling (biopsy) by puncture.

Since the puncture needle 5a of the puncture needle apparatus 5 has an outside diameter D1 smaller than 2 mm and a total length L1 of about 400 mm as described earlier, when the surgeon grips the grasping portion 5b on a proximal end side of the puncture needle 5a as shown in step S6, a distal end of the puncture needle 5a becomes very prone to generate vibration.

Consequently, the surgeon grips the grasping portion 5b in a gripping state in which the surgeon cannot completely stabilize the hand gripping the grasping portion 5b, i.e., in a state in which so-called shaking movements are unavoidable, and so the distal end side of the puncture needle 5a goes into a vibrational state in which the distal end side of the puncture needle 5a vibrates as if resonating at a frequency close to that of the shaking movements.

An operation in which the surgeon inserts the distal end side of the puncture needle 5a into the channel opening portion 4b with a small inner diameter (e.g., about 2 to 3 mm) alone without using the treatment instrument insertion auxiliary 6 according to the present embodiment becomes difficult to perform in a short time due to vibration such as described above. That is, the operation of inserting the distal end side of the puncture needle 5a into the channel opening portion 4b become difficult because the distal end side of the puncture needle 5a vibrates with an amplitude far greater than the inner diameter of the channel opening portion 4b.

According to the present embodiment, the treatment instrument insertion auxiliary 6 is provided with the receiving portion 53 having a large area so as to be able to suppress shaking or vibration such as described above (hereinafter simply referred to as vibration).

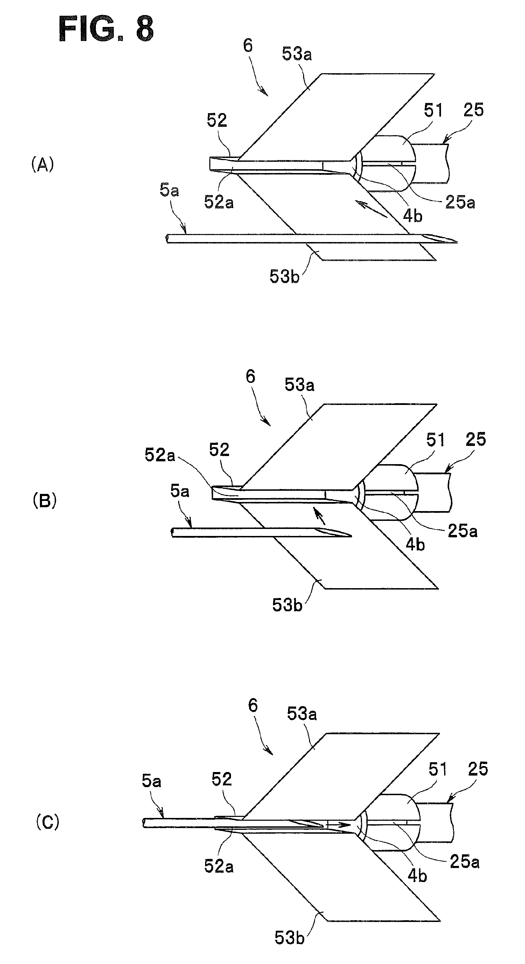

As shown in step S7, the surgeon places the vibrating distal end side of the puncture needle 5a in contact with a plane of the receiving portion 53. Fig. 8(A) shows how the surgeon places the distal end side of the puncture needle 5a in contact with a plane of one 53b of the plate members making up the receiving portion 53.

By placing the distal end side of the puncture needle 5a in contact with the plane of the receiving portion 53, it is possible to suppress vibration of the distal end side of the puncture needle 5a. That is, as shown in step S8, the vibration of the distal end side of the puncture needle 5a goes into a suppressed state.

Note that since the receiving portion 53 is made up of the two plate members 53a and 53b so as to have a larger area, even if the distal end side of the puncture needle 5a is vibrating, the surgeon can place the distal end side of the puncture needle 5a in contact with some part of the large-area receiving portion 53. When the distal end side of the puncture needle 5a is vibrating, the receiving portion 53 absorbs vibration energy and thereby suppresses the vibration via a receiving portion-side abutting portion abutted by the vibrating portion.

After the vibration of the distal end side of the puncture needle 5a is suppressed, as shown in step S9, the surgeon causes the distal end side of the puncture needle 5a to slip or slidingly move toward the guide path 52 while keeping the distal end side of the puncture needle 5a in contact with the plane of the receiving portion 53. Incidentally, for example, as indicated by chain double-dashed lines in Fig. 5(C), in order to make it possible to more effectively suppress the vibration of the distal end side of the puncture needle 5a, a vibration-absorbing member 60 of rubber or the like with a high capability to absorb vibration (damp vibration) may be applied, for example, as a thin film or with an appropriate thickness to surfaces of the plate members 53a and 53b making up the receiving portion 53. This may also be applied to other embodiments and the like described later. Note that if the thickness is increased, when placed in contact with the distal end side of the puncture needle 5a, the plate members 53a and 53b will get deformed and abut a portion on the distal end side of the puncture needle 5a over a large area, further enhancing the capability to absorb vibration.

Fig. 8(B) shows a state in which the distal end side of the puncture needle 5a is in the process of being moved toward the guide path 52 from the state of Fig. 8(A). By going through the state of Fig. 8(B), the surgeon further moves the distal end side of the puncture needle 5a toward the guide path 52 and continues a sliding movement until the distal end side of the puncture needle 5a abuts an inner wall of the guide groove (guiding groove) 52a of the guide path 52. Then, as shown in step S10, the distal end side of the puncture needle 5a can be housed in the guide groove 52a of the guide path 52.

Fig. 8(C) shows how the distal end side of the puncture needle 5a is housed in the guide groove 52a of the guide path 52 as a result of an action in step S9.

As shown in step S11, the surgeon can insert the distal end side of the puncture needle 5a into the channel 4a through the channel opening portion 4b by moving or pushing in the distal end side of the puncture needle 5a housed in the guide groove 52a forward.

By pushing in the puncture needle 5a to (or to near) a position where the proximal end of the puncture needle 5a abuts the connecting tube 25a, it is possible to set the distal end of the puncture needle 5a at a position near the channel distal end opening portion 4c.

As shown in step S12, the surgeon can verify the distal end side of the puncture needle 5a (e.g., a state indicated by a chain double-dashed line in Fig. 1) with the ultrasound tomographic image 9a. Then, as shown in step S13, with the distal end side of the puncture needle 5a set at a position on the near side of the biopsy site where a biopsy is going to be taken, the surgeon causes the distal end side of the puncture needle 5a to protrude, pierces the biopsy site with the distal end of the puncture needle 5a, and performs tissue sampling (biopsy) by housing the tissue in the recess 5e on the distal end side of the puncture needle 5a.

After the biopsy, the surgeon draws the puncture needle 5a out of the channel 4a as shown in step S 14. As shown in step S 15, the surgeon determines whether to further perform a biopsy.

If a biopsy is to be performed further, the surgeon sets the distal end side of the ultrasound probe 3 to a next biopsy site as shown in step S16. Then, by repeating step S5 and subsequent operations using a new puncture needle apparatus 5, a biopsy can be performed, for example, at a biopsy site different from the previous time. After repeating multiple biopsies to perform the biopsies at plural desired biopsy sites in this way, the operation of Fig. 7 is finished.

As described above, according to the present embodiment, since vibration is suppressed easily by placing the vibrating distal end side of the puncture needle 5a in contact with the receiving portion 53 and the distal end side of the puncture needle 5a is inserted into the channel 4a by simple actions with the vibration suppressed, even when biopsies are performed at plural locations, the surgeon can perform the biopsies smoothly by inserting the distal end side of the puncture needle 5a into the channel 4a by himself/herself in a short time.

Although in the above description, it is assumed that the adapter member 35 is not used, when the puncture needle 5a of the puncture needle apparatus 5 is passed through the channel 4a of the optical scope 4 using the adapter member 35, the distal end side of the puncture needle 5a may be set at such a position in the channel distal end opening portion 4c that is suitable for biopsy.

Next, description will be given of an operation of performing a biopsy by using the adapter member 35 and observing the ultrasound tomographic image 9a.

As shown in Fig. 4, the connection portion 41a of the adapter member 35 is attached to the connecting tube 25a and the connection portion 51 of the treatment instrument insertion auxiliary 6 is attached to the connection portion 42a of the adapter member 35 (the attached state is indicated by chain double-dashed lines).

In this state, the distal end side of the puncture needle 5a of the puncture needle apparatus 5 is inserted into the treatment instrument insertion auxiliary 6 from behind as described with reference to Fig. 7.

Subsequently, with a front end face of the grasping portion 5b at the proximal end of the puncture needle 5a set at such a position as to abut an opening portion at a proximal end of the guide path 52, a total length of the adapter member 35 (more specifically, a distal end position of the outer tube 42 capable of sliding movement with respect to the inner tube 41) is adjusted to a state (state indicated by chain double-dashed lines in Fig. 1) in which the distal end of the puncture needle 5a protrudes, for example, slightly from the channel distal end opening portion 4c.

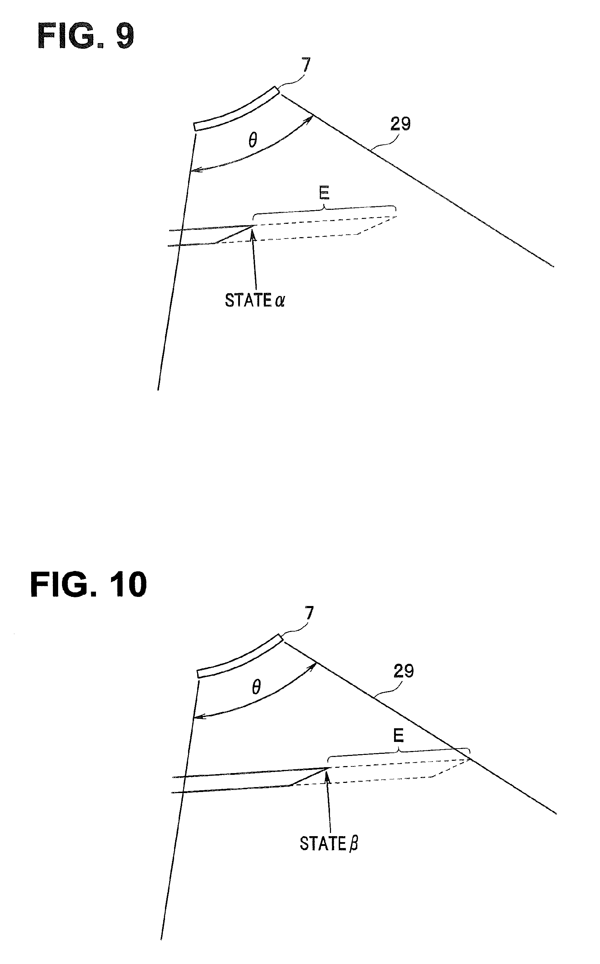

Also, on an observed image based on an ultrasound tomographic image 9a, the state in which the distal end of the puncture needle 5a protrudes, for example, slightly from the channel distal end opening portion 4c is a state such as shown in Fig. 9.

To create such a state, the outer tube 42 capable of sliding movement with respect to the inner tube 41 of the adapter member 35 is adjusted in length and fixed, for example, near the position indicated by α in Fig. 4 with the fixing screw 44 in a set state after the adjustment. On the ultrasound tomographic image 9a, this state corresponds to a set state (simply referred to as state α) such as shown in Fig. 9, and when the puncture needle 5a made up of the inner needle 5c and outer needle tube 5d (shown in Fig. 3) is caused to protrude by a protruding length E by automatic biopsy (as indicated by broken lines in Fig. 9), the distal end of the puncture needle 5a is located within the ultrasound scan field 29.

On the other hand, when the outer tube 42 is located at position P and the fixing screw 44 is kept tight at a position corresponding to β, the distal end of the puncture needle 5a is in a set state (state β) shown in Fig. 10. When the outer tube 42 is at position β, if the puncture needle 5a is caused to protrude by a protruding length E by automatic biopsy (as indicated by broken lines in Fig. 10), the distal end barely falls within the ultrasound scan field 29. In other words, the circumferential groove 45 is provided at a fixed position which depends on the length of the needle and the ultrasound scan field 29.

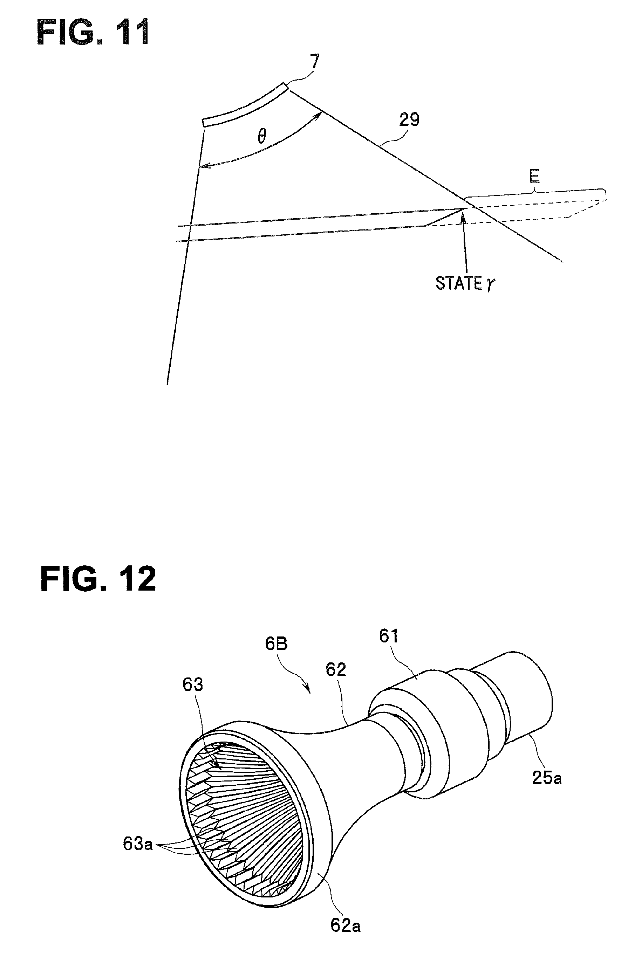

Also, when the outer tube 42 is located at position γ and the fixing screw 44 is kept tight at a position corresponding to γ, the distal end of the puncture needle 5a is in a set state (state γ) shown in Fig. 11. When the outer tube 42 is at position γ, if the puncture needle 5a is caused to protrude by a protruding length E by automatic biopsy (as indicated by broken lines in Fig. 11), the distal end falls outside the ultrasound scan field 29. In other words, only by removing the movement restriction member 46 from the circumferential groove 45, the surgeon can puncture a site outside the ultrasound scan field 29.

By setting the total length of the adapter member 35 such that lengths of the channel 4a and the puncture needle 5a will be appropriate using the adapter member 35 in this way, it is possible to take a biopsy from the biopsy site by automatic biopsy in a short time.

As described above, according to the present embodiment, the distal end side of the treatment instrument can be guided smoothly in the central axis direction of the channel opening portion 4b serving as a treatment instrument insertion opening portion of the channel 4a by a surgeon alone by simple actions. In other words, the puncture needle 5a of the puncture needle apparatus 5 as a treatment instrument can be inserted into the channel 4a in a short time. Consequently, even when biopsies are performed at plural locations, the biopsies can be performed at the plural locations in a short time.

### (Second Embodiment)

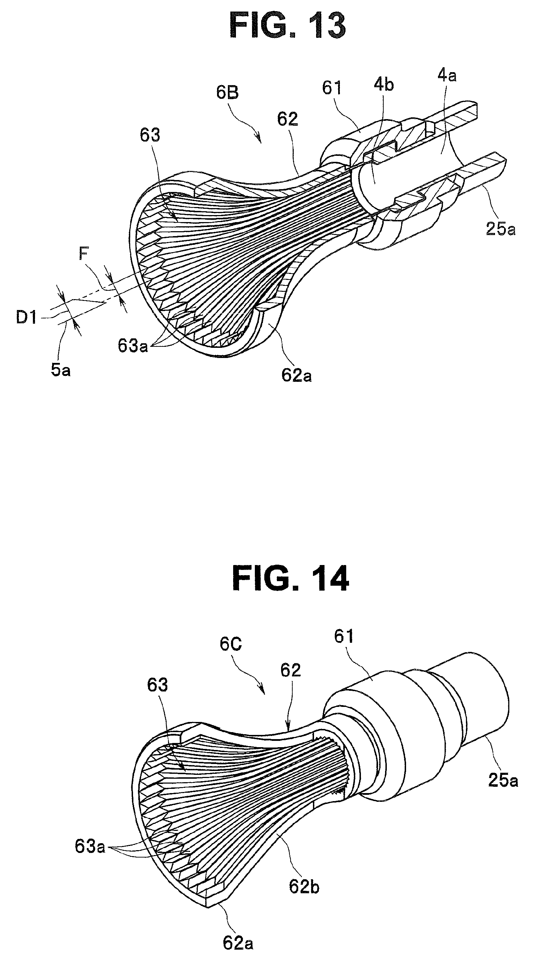

Next, a second embodiment of the present invention will be described with reference to Fig. 12. Fig. 12 shows an appearance of a treatment instrument insertion auxiliary 6B according to a second embodiment as connected (attached) to the connecting tube 25a and Fig. 13 shows an internal structure by cutting away part of an outer circumferential face.

The treatment instrument insertion auxiliary 6B allows a treatment instrument guide member (or treatment instrument guide unit) 62 made of a funnel-shaped metal member to be detachably connected (attached) to the connecting tube 25a of the channel 4a via a resilient coupling member (or connecting member) 61 of rubber or the like. By abutting a rear end face of the connecting tube 25a (i.e., an end face of the channel opening portion 4b), a distal end face of the treatment instrument guide member 62 is connected to the connecting tube 25a by the coupling member 61.

The treatment instrument guide member 62 has a ring-shaped distal end side opening portion whose distal end is equal in inner diameter to the channel opening portion 4b, and the treatment instrument guide member 62 forms a funnel shape, with its inner diameter and outside diameter spreading into a circular conical shape from the distal end side opening portion toward a rear end side of the treatment instrument guide member 62. An opening portion at a rear end forms a treatment instrument insertion opening portion portion (simply referred to as an insertion opening portion) 62a.

Also, according to the present embodiment, on an entire inner circumferential face of the treatment instrument guide member 62 spreading into a funnel shape, plural guide grooves (or guide ridges) 63a shaped, for example, triangular in cross-section and extended out along a longitudinal direction of the treatment instrument guide member 62 are provided densely in a circumferential direction, forming a guide path (or a guide unit) 63 adapted to guide the distal end side of the puncture needle 5a in the central axis direction of the channel 4a. The guide groove 63a is not limited to a triangular cross-section, and may be a U-shaped recess. Besides, the guide grooves 63a may form a concavo-convex portion of another cross-sectional shape. Note that according to the present embodiment, wall surfaces of the guide grooves 63a making up the guide path 63 functions as a receiving portion adapted to suppress vibration of the distal end side of the puncture needle 5a by being placed in contact with the distal end side of the puncture needle 5a. Therefore, according to the present embodiment, the guide path 63 which combines the function of the receiving portion forms a treatment instrument guide unit (which includes the receiving portion and guide path).

The guide grooves 63a making up the guide path 63 are formed so as to extend along a longitudinal direction so that circumferential position will not change.

For example, when a bottom portion of an arbitrary guide groove 63a is cut along a cutting plane passing through the central axis of the channel 4a, the bottom portion of the guide groove 63a is cut in such a way that the circumferential position will not change at any position along the longitudinal direction.

Also, as shown in Fig. 13, near the insertion opening portion 62a on an inlet side of the guide path 63, a groove width F of the guide groove 63a is set to a size smaller than the outside diameter D1 of the puncture needle 5a. Consequently, unless the distal end side of the puncture needle 5a abuts the wall surface of a guide groove 63a at a large angle, before a cutting edge at the distal end of the puncture needle 5a hits the wall surface of the guide groove 63a, a tubular outer circumferential face on a rear end side of the cutting edge abuts the wall surface so as to be able to prevent the cutting edge from being damaged by hitting the wall surface.

That is, again according to the present embodiment, by inserting the distal end side of the puncture needle 5a into a wide-open inner side of the treatment instrument guide member 62 and causing the distal end side of the puncture needle 5a to abut any of the wall surfaces making up the large number of the guide grooves 63a formed on the entire inner circumferential face of the treatment instrument guide member 62, the surgeon can suppress vibration of the distal end side of the puncture needle 5a. Subsequently, with the distal end side of the puncture needle 5a abutting the guide groove 63a, by pushing out or moving the distal end side of the puncture needle 5a forward (toward the far side), the surgeon can move the distal end side of the puncture needle 5a along the longitudinal direction of the abutted guide groove 63a and insert the distal end side into the channel opening portion 4b.

Again, according to the present embodiment, the guide path 63 serving the function of a receiving portion placed in contact with the distal end side of the puncture needle 5a is configured to be wide open.

Thus, by inserting the distal end side of the puncture needle 5a into the insertion opening portion 62a and placing the distal end side in contact with the wall surface of any of the guide grooves 63a in an inner circumferential face of the insertion opening portion 62a, the surgeon can suppress the vibration of the distal end side of the puncture needle 5a and subsequently move the distal end side of the puncture needle 5a toward the far side, thereby inserting the distal end side of the puncture needle 5a into the channel opening portion 4b by simple actions in a short time.

Note that although the insertion opening portion 62a is formed into a ring shape in the example shown in Fig. 12, part of a funnel-shaped outer circumferential face portion may be cut away along a longitudinal direction except for a portion at the distal end side connected to the channel tube 25 of the treatment instrument guide unit member 62. More specifically, as shown in Fig. 14, a conceivable treatment instrument insertion auxiliary 6C is configured such that a lateral opening portion 62b which opens to a side is provided to allow the distal end side of the puncture needle 5a to be inserted into the channel opening portion 4b by being placed in contact with a guide groove 63a of the guide path 63 from a side as well.

Whereas with the configuration in Fig. 12, the distal end side of the puncture needle 5a needs to be inserted through a circular opening of the insertion opening portion 62a formed in the longitudinal direction of the guide path 63, with the configuration in Fig. 14, the distal end side of the puncture needle 5a can also be inserted into the guide path 63 from the side where the lateral opening portion 62b (as a lateral opening portion orthogonal to the central axis direction of the channel 4a) opens.

This allows the surgeon to select an insertion direction of the distal end side of the puncture needle 5a from a wider range of directions and perform an operation of inserting the distal end side of the puncture needle 5a in a direction considered to enable easier insertion. Thus, the present variation improves operability compared to the configuration of Fig. 12. Note that although the funnel-shaped outer circumferential face portion is partially cut away by being nearly bisected along the longitudinal direction as shown in Fig. 14, the cutaway is not limited to such a type, and the outer circumferential face portion may be cut, for example, by forming a slope at an appropriate angle to the longitudinal direction.

### (Third Embodiment)

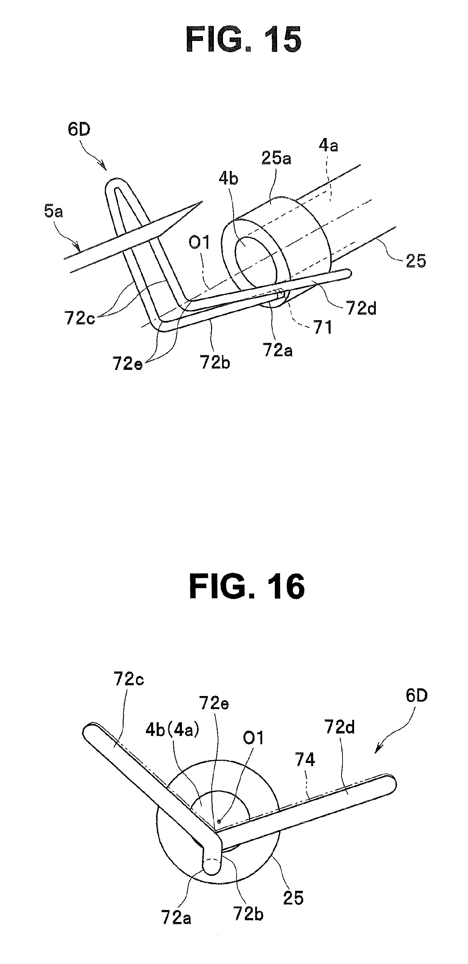

Next, a third embodiment of the present invention will be described. Fig. 15 shows a configuration of a treatment instrument insertion auxiliary 6D according to the third embodiment of the present invention in the form of a perspective view. Also, Fig. 16 shows a rear view of the treatment instrument insertion auxiliary 6D as viewed from behind the treatment instrument insertion auxiliary 6D along the central axis of the channel 4a, with the treatment instrument insertion auxiliary 6D fixed to an end face of the channel tube 25.

The treatment instrument insertion auxiliary 6D according to the present embodiment is configured with a wire-shaped member (referred to as wire or wire member). A hole portion 71 used to fix the treatment instrument insertion auxiliary 6D is provided in the end face of the channel tube 25 and the treatment instrument insertion auxiliary 6D can be detachably fixed (connected) by press-fitting a fixing end portion 72a of the treatment instrument insertion auxiliary 6D into the hole portion 71.

The treatment instrument insertion auxiliary 6D includes an extending portion 72b extended out rearward substantially parallel to the central axis 01 of the channel tube 25 (or channel 4a) from the fixing end portion 72a, a first receiving portion 72c formed by being extended out from an end portion of the extending portion 72b in a direction perpendicular to the central axis 01 and being folded into a U-shape (or V-shape) at the extended position, and a second receiving portion 72d formed by being bent from an end portion of the first receiving portion 72c into an L-shape so as to extend out in a direction perpendicular to the central axis 01.

As shown in the rear view of Fig. 16, the treatment instrument insertion auxiliary 6D formed in this way includes the receiving portions 72c and 72d extended out in a direction approximately orthogonal to the central axis 01, forming a V-shape on an extension of the central axis 01 of the channel 4a as well as includes a guide unit (or guide path) 72e with a (bending) boundary between the receiving portions 72c and 72d formed near the extension of the central axis 01 of the channel 4a. Therefore, according to the present embodiment, a treatment instrument guide unit which combines functions of a receiving portion and guide unit is formed by the receiving portions 72c and 72d. Also, the guide unit (or guide path) 72e is structured to be able to abut the puncture needle 5a at two locations near the extension of the central axis 01 of the channel 4a. By abutting at two locations, the guiding portion can guide the act of inserting the distal end side of the puncture needle 5a into the channel opening portion 4b. Also, the guide unit 72e actually has a V-shape (in a rear view seen from a direction of the extension of the central axis 01 of the channel 4a), and thus serves a function of a restricting portion adapted to restrict the moving direction of the distal end side of the puncture needle 5a.

According to the present embodiment, by placing the distal end side of the puncture needle 5a in contact with the first receiving portion 72c, for example, as shown in Fig. 15, the vibration (or shaking) of the distal end side of the puncture needle 5a can be suppressed, and subsequently by moving the distal end side of the puncture needle 5a in contact with the first receiving portion 72c to the side of the guide unit 72e at the bending boundary, the distal end side of the puncture needle 5a can be guided in a direction closer to the central axis 01 of the channel 4a. Thus, once the distal end side of the puncture needle 5a is set to a direction of the guide unit 72e, by moving the distal end side of the puncture needle 5a forward, the distal end side of the puncture needle 5a can be easily inserted into the channel 4a.

As with the first embodiment or the second embodiment, the present embodiment can provide a treatment instrument insertion auxiliary which lends itself to low-cost manufacturing and allows a single surgeon to easily guide the distal end side of the treatment instrument in the central axis direction of the channel opening portion 4b serving as a treatment instrument insertion opening portion of the channel 4a without requiring an assistant. Again, in the present embodiment, a vibration-absorbing member 74 of rubber or the like with a high capability to absorb vibration may be applied as a thin film, for example, as indicated by chain double-dashed lines in Fig. 16, for example, to surfaces of the receiving portions 72c and 72d formed of a wire member. Note that the treatment instrument insertion auxiliary 6D may be produced using, for example, a tube-shaped vibration-absorbing member covering an outer circumferential face of the wire member instead of the thin-film vibration-absorbing member. Note that the vibration-absorbing member 74 may be applied to a receiving portion 75 in the following variation.

In the treatment instrument insertion auxiliary 6D according to the present embodiment, when viewed from a rear side, the receiving portions 72c and 72d are formed into a substantially V-shape using a wire member as shown in Fig. 16. In contrast, the receiving portion may be formed into a spiral shape as described below instead of the V-shape. In other words, the receiving portion may be constructed using a wire formed into a V-shape or spiral shape in a direction approximately orthogonal to the axis on an extension of a central axis of the treatment instrument channel or a treatment instrument guide unit which combines a receiving portion and a guide unit may be constructed.

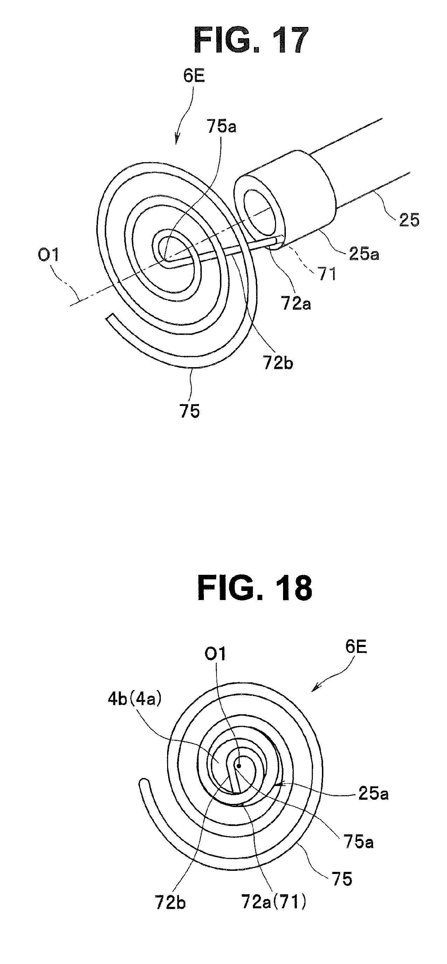

Fig. 17 shows a treatment instrument insertion auxiliary 6E according to a variation of the third embodiment of the present invention in the form of a perspective view. Fig. 18 shows a rear view of the treatment instrument insertion auxiliary 6E viewed from a rear side. The treatment instrument insertion auxiliary 6E has a spiral-shaped receiving portion 75 at a rear end of the extending portion 72b.

A boundary between the rear end of the extending portion 72b and the receiving portion 75 formed into a spiral shape constitutes a guide unit 75a. In other words, a neighborhood of a center of a spiral in the receiving portion 75 constitutes the guide unit 75a located on an extension of the central axis 01 of the channel 4a.

The surgeon performs the act of placing the distal end side of the puncture needle 5a in contact with any part of the spiral-shaped wire making up the receiving portion 75. By placing the distal end side of the puncture needle 5a in contact with any part of the spiral-shaped portion making up the receiving portion 75, it is possible to suppress vibration of the distal end side of the puncture needle 5a.

Subsequently, by moving the distal end side of the puncture needle 5a to a center side of the spiral, the distal end side of the puncture needle 5a can be set on the guide unit 75a at the center of the spiral located on the extension of the central axis 01 of the channel 4a, and by moving the distal end side of the puncture needle 5a along the guide unit 75a, the distal end side of the puncture needle 5a can be inserted into the channel 4a. The present variation provides advantages similar to those of the third embodiment and lends itself to low-cost manufacturing.

Note that embodiments configured by combining parts of the embodiments and the like described above are also included in the present invention. Also, the present invention can be used not only to subserve insertion of a treatment instrument such as the puncture needle apparatus 5 equipped with a linear puncture needle 5a into a treatment instrument channel when the axis is a straight line as shown in Fig. 1 and the like, but also to subserve insertion of a non-linear treatment instrument equipped with a flexing portion. Also, as a treatment instrument insertion auxiliary apparatus (or treatment instrument insertion auxiliary system or endoscope apparatus) which includes a treatment instrument insertion auxiliary according to an original claim, a configuration which further includes an endoscope (rigid endoscope) and the like provided with a rigid insertion portion made up of an optical scope 4 equipped with a treatment instrument channel can also constitute a claim.

Also, in the present invention, one or more components may be added as appropriate on the basis of a configuration described in an independent claim. Also, if a description in an original dependent claim at the time of application differs from a description literally disclosed in the original specification, the wording of the original specification may be substituted for the wording of the original dependent claim. Also, a claim cited in a dependent claim may be changed to another claim as long as consistency is maintained. Also, the present invention discloses treatment instrument insertion methods whose details are described in appendices below.

[Appendix 1] A treatment instrument insertion method for passing an elongated treatment instrument through a treatment instrument channel formed along an axis, comprising:
a first step of connecting or fixing one end of a treatment instrument insertion auxiliary adapted to subserve insertion of the treatment instrument to an insertion port provided at a proximal end of the treatment instrument channel and used to insert a distal end side of the treatment instrument;
a second step of placing the distal end side of the treatment instrument in contact with a receiving portion provided on the treatment instrument insertion auxiliary and adapted to suppress vibration of the distal end side of the treatment instrument;
a third step of moving the the distal end side of the treatment instrument whose vibration is suppressed by the second step from a position where the distal end side of the treatment instrument is placed in contact with the receiving portion in a direction of an extension of a central axis of the treatment instrument channel, making the distal end side of the treatment instrument concentric with the central axis; and
a fourth step of moving the distal end side of the treatment instrument to a side of the insertion port along the direction of the extension after the distal end side of the treatment instrument is set in the direction of the extension of the central axis by the third step and inserting a distal end of the treatment instrument into the insertion port facing forward in the direction of the extension.

[Appendix 2] The treatment instrument insertion method according to appendix 1, wherein: the treatment instrument insertion auxiliary includes a guide groove coupled to the receiving portion and extended out in a direction in which guide groove becomes concentric with the central axis of the treatment instrument channel and
the third step inserts the distal end side of the treatment instrument positioned in contact with the receiving portion into the guide groove while maintaining the contact and thereby moves the distal end side of the treatment instrument in the direction of the extension on which the distal end side of the treatment instrument becomes concentric with the central axis of the treatment instrument channel.

[Appendix 3] The treatment instrument insertion method according to appendix 1, wherein: the second step places the distal end side of the treatment instrument in contact with a receiving portion formed by a spiral wire or a V-shaped wire whose center is located on the extension of the central axis of the treatment instrument channel and thereby suppresses the vibration of the distal end side of the treatment instrument placed in contact with the receiving portion.

[Appendix 4] The treatment instrument insertion method according to appendix 2, wherein: the receiving portion includes two planes which spreads into a V-shape in cross-section from an open edge of the guide groove; and the second step places the distal end side of the treatment instrument in contact with either one of the two planes and the plane placed in contact suppresses the vibration of the distal end side of the treatment instrument.

[Appendix 5] The treatment instrument insertion method according to appendix 2, wherein: the receiving portion includes two planes which spreads into a V-shape in cross-section from an open edge of the guide groove; and a vibration-absorbing portion adapted to absorb vibration and provided on each of the two planes, wherein the second step places the distal end side of the treatment instrument in contact with either one of the two planes, and causes the vibration-absorbing portion to absorb the vibration of the distal end side of the treatment instrument.

[Appendix 6] The treatment instrument insertion method according to appendix 1, wherein: the receiving portion includes a V-shaped portion extended out in a direction substantially orthogonal to the central axis from a position on the extension of the central axis of the the treatment instrument channel; and a vibration-absorbing portion adapted to absorb vibration and provided on a surface of the V-shaped portion, wherein the second step places the distal end side of the treatment instrument in contact with the V-shaped portion and causes the vibration-absorbing portion provided on the surface of the V-shaped portion to absorb the vibration of the distal end side of the treatment instrument placed in contact.

The present application is based upon and claims the benefit of priority from Japanese Patent Application No. 2012-166132, filed in Japan on July 26, 2012, the entire contents of which are incorporated in the specification, claims, and drawings herein by reference.

## Claims

1. A treatment instrument insertion auxiliary used to subserve passage of an elongated treatment instrument into a treatment instrument channel by being connected to an endoscope provided with a rigid insertion portion which includes the treatment instrument channel adapted to allow passage of the treatment instrument and a treatment instrument channel opening portion formed at a proximal end of the treatment instrument channel and used to insert the treatment instrument, the treatment instrument insertion auxiliary comprising
a treatment instrument guide unit adapted to guide a distal end side of the treatment instrument in a central axis direction of the treatment instrument channel opening portion by being placed in contact with the distal end side of the treatment instrument.

2. The treatment instrument insertion auxiliary according to claim 1, wherein the treatment instrument guide unit includes:
a receiving portion adapted to suppress vibration of the distal end side of the treatment instrument by being placed in contact with the distal end side of the treatment instrument; and
a guide path adapted to guide the distal end side of the treatment instrument placed in contact with the receiving portion in the central axis direction of the treatment instrument channel opening portion.

3. The treatment instrument insertion auxiliary according to claim 2, wherein the guide path includes a guiding portion adapted to guide a moving direction of the distal end side of the treatment instrument along a direction parallel to the central axis within a distance equal to a radius of the treatment instrument channel opening portion from a central axis of the treatment instrument channel opening portion by being abutted by the distal end side of the treatment instrument at at least two locations.

4. The treatment instrument insertion auxiliary according to claim 2, wherein:
the receiving portion is made up of two plate members having a plane, one side each of the two plate members which face each other in close vicinity are combined so as to form the guide path, and the one side each of the two plate members extends out substantially in parallel to the central axis of the treatment instrument channel in an inner diameter of a hollow portion of the treatment instrument channel; and
the guide path is formed on an extension of the central axis so as to be substantially concentric with the central axis of the treatment instrument channel.

5. The treatment instrument insertion auxiliary according to claim 3, wherein:
the receiving portion is made up of two plate members having a plane, one side each of the two plate members which face each other in close vicinity are combined so as to form the guide path, and the one side each of the two plate members extends out substantially in parallel to the central axis of the treatment instrument channel in an inner diameter of a hollow portion of the treatment instrument channel; and
the guide path is formed on an extension of the central axis so as to be substantially concentric with the central axis of the treatment instrument channel.

6. The treatment instrument insertion auxiliary according to claim 2, further comprising a connection portion used to detachably connect to the treatment instrument channel opening portion.

7. The treatment instrument insertion auxiliary according to claim 2, wherein the treatment instrument guide unit has a funnel shape made up of a first opening portion connected to the treatment instrument channel opening portion and a second opening portion larger in bore than the first opening portion.

8. The treatment instrument insertion auxiliary according to claim 2, further comprising a slide unit configured to be advanceable and retractable along an axial direction of the treatment instrument channel and variable in total length.

9. The treatment instrument insertion auxiliary according to claim 8, wherein in the slide unit, one end of the slide unit is detachably connected to the treatment instrument channel opening portion and another end of the slide unit is detachably connected with a connection portion at a proximal end of the treatment instrument guide unit.

10. The treatment instrument insertion auxiliary according to claim 7, wherein; the treatment instrument guide unit having the funnel shape is formed on an entire inner circumferential face of the funnel shape, and provided with a plurality of concavo-convex portions along a longitudinal direction of the funnel shape to guide the distal end side of the treatment instrument; and each concave portion of the concavo-convex portions is smaller than an outside diameter of the distal end side of the treatment instrument.

11. The treatment instrument insertion auxiliary according to claim 2, wherein:
the treatment instrument guide unit has a V-shaped portion or a spiral-shaped portion formed using a wire at a position on an extension of the central axis of the treatment instrument channel, the V-shaped portion extending out in a V-shaped manner and the spiral-shaped portion extending out in a spiral-shaped manner, in a direction approximately orthogonal to the central axis; and
the V-shaped portion or the spiral-shaped portion forms the receiving portion while the position on the extension of the central axis forms the guide path.

12. The treatment instrument insertion auxiliary according to claim 3, wherein:
the treatment instrument guide unit has a V-shaped portion formed using a wire at a position on an extension of the central axis of the treatment instrument channel, the V-shaped portion extending out in a V-shaped manner in a direction approximately orthogonal to the central axis; and
the V-shaped portion forms the receiving portion and two positions on the extension of the central axis form the guide path.

13. The treatment instrument insertion auxiliary according to claim 2, wherein the receiving portion has a vibration-absorbing member formed on a surface of the receiving portion, the vibration-absorbing member being adapted to absorb vibration of the distal end side of the treatment instrument when placed in contact with the distal end side of the treatment instrument.

14. The treatment instrument insertion auxiliary according to claim 3, wherein the receiving portion has a vibration-absorbing member formed on a surface of the receiving portion, the vibration-absorbing member being adapted to absorb vibration of the distal end side of the treatment instrument when placed in contact with the distal end side of the treatment instrument.
